# EUROPEAN PATENT APPLICATION

(11) **EP 4 631 532 A1**
(43) Date of publication of application: **15.10.2025**
(21) Application number: 23914576.6
(22) Date of filing: 28.12.2023
(51) Int. Cl.: A61L 24/00, C08F 261/00

(54) **PREPARATION METHOD FOR EMBOLIZATION MICROSPHERES**

(30) Priority: 05.01.2023 CN 202310011511
(71) Applicant: Shanghai Intervascular MedTech Co., Ltd., Shanghai 201318 (CN)
(72) Inventor: XU, Qian, Shanghai 201318 (CN); NIU, Haifeng, Shanghai 201318 (CN); GAO, Lingyue, Shanghai 201318 (CN); WANG, Jinyao, Shanghai 201318 (CN)
(74) Representative: Murgitroyd & Company
(86) International application number: PCT/CN2023/142893
(87) International publication number: WO 2024/146444

(57) **Abstract**

A preparation method for embolization microspheres, comprising the following steps: grafting an unsaturated bond onto a water-soluble polymer to prepare a modified polymer; mixing the modified polymer, a water-soluble monomer, a water-soluble initiator and water to prepare a water-phase solution, the water-soluble monomer containing an unsaturated bond; mixing an oil-soluble stabilizer with an oily solvent to prepare an oil-phase solution; at a stirring rotating speed of about 500 r/min to about 1200 r/min and in an axial flow stirring mode, adding the water-phase solution to the oil-phase solution to form a water-in-oil emulsification system; and adding a catalyst to the emulsification system under a protective atmosphere while stirring, and subjecting the unsaturated bond in the modified polymer and the unsaturated bond in the water-soluble monomer to free radical polymerization, so as to prepare the embolization microspheres.

## Description

### RELATED APPLICATIONS

This application claims priority to Chinese patent application No. 202310011511.2, entitled "PREPARATION METHOD FOR EMBOLIZATION MICROSPHERES" filed on January 05, 2023, the content of which is hereby incorporated by reference in its entirety.

### TECHNICAL FIELD

The present application relates to the field of embolic agents, in particular to a method for preparing embolic microspheres.

### BACKGROUND

In recent years, with the development of minimally invasive interventional techniques, interventional oncology has been applied in the field of tumor treatment and has become an important method for treating cancer, especially playing an extremely critical role in the treatment of hypervascular tumors, such as liver cancer, kidney cancer, and uterine fibroids. The principle of interventional embolization is to deliver embolic materials to the blood-supplying vessels of the tumor through a microcatheter, a microwire, etc. under the guidance of X-ray fluoroscopy, thereby blocking the blood supply to the tumor and causing the tumor cells to atrophy and necrose due to nutrient deprivation, ultimately achieving a therapeutic goal.

The interventional embolization heavily relies on selecting an appropriate embolic material for blocking the blood supply to the tumor. Polyvinyl alcohol embolic microspheres have been widely used in clinical embolization due to their excellent biocompatibility, regular shape, uniform size, superior compression-rebound performance, and ability to carry chemotherapeutic drugs. The embolization of tumor vessels at different anatomical sites requires microspheres with varying particle size ranges. Currently, the commercially available microspheres predominantly have particle sizes ranging from 100 µm to 300 µm, 300 µm to 500 µm, and 500 µm to 700 µm, which can achieve desired therapeutic effects in most embolization procedures. However, for specific tumors and cases, small-sized embolic microspheres offer unique advantages. For example, for the prevalent hypovascular liver metastases including those from colorectal and breast cancers, small-sized microspheres can penetrate deeper into the tumor vasculature, reducing the likelihood of residual perfusion through collateral vessels and leads to severer necrosis. Nevertheless, the conventional preparation methods of the small-sized (less than 100 µm) embolic microspheres suffer from low yield and poor appearance, significantly hindering and limiting the development and clinical application of the small-sized embolic microspheres.

### SUMMARY

In view of this, some embodiments of the present application provide a method for preparing embolic microspheres.

A method for preparing embolic microspheres includes the following steps:
grafting an unsaturated bond onto a water-soluble polymer to prepare a modified polymer, wherein a mass ratio of the water-soluble polymer to a grafting reagent is substantially 1:(0.016 to 0.07), the water-soluble polymer contains a hydroxyl group, and the grafting reagent contains the unsaturated bond and an acetal group;
mixing the modified polymer, a water-soluble monomer, a water-soluble initiator, and water to prepare an aqueous phase solution, wherein the water-soluble monomer contains an unsaturated bond and an ionic group, and a mass ratio of the water-soluble monomer to the water-soluble polymer is substantially (0.24 to 1.16):1;
mixing an oil-soluble stabilizer with an oily solvent to prepare an oil phase solution;
adding the aqueous phase solution to the oil phase solution under axial flow stirring at a stirring rotation speed in a range from about 500 revolutions/minute (r/min) to about 1200 r/min to form a water-in-oil emulsion system; and
adding a catalyst to the emulsion system under stirring in a protective atmosphere to initiate free radical polymerization of the unsaturated bond of the modified polymer and the unsaturated bond of the water-soluble monomer, thereby forming the embolic microspheres.

In an embodiment, in the step of adding the aqueous phase solution to the oil phase solution, the stirring rotation speed is in a range from about 800 r/min to about 1000 r/min.

In an embodiment, in the step of adding the aqueous phase solution to the oil phase solution, a stirring paddle is adopted and includes a four-blade metal stirring paddle.

In an embodiment, a mass ratio of the catalyst to the modified polymer is substantially (0.533 to 4.8):100.

In an embodiment, the catalyst is tetramethylethylenediamine, a persulfate, or a combination thereof.

In an embodiment, the free radical polymerization is performed at a temperature in a range from about 45 °C to about 65 °C for a time period in a range from about 2 hours to about 8 hours.

In an embodiment, in the step of adding the catalyst to the emulsion system to initiate the free radical polymerization of the unsaturated bond of the modified polymer and the unsaturated bond of the water-soluble monomer, a stirring rotation speed is in a range from about 150 r/min to about 400 r/min.

In an embodiment, the step of grafting the unsaturated bond onto the water-soluble polymer includes: dissolving the water-soluble polymer in water, adjusting pH to acidic, reacting the water-soluble polymer with the grafting reagent at a temperature in a range from about 40 °C to about 50 °C for a time period in a range from about 3 hours to about 24 hours, adjusting the pH to neutral or alkaline after the reaction, and performing dialysis purification to prepare a modified polymer-containing solution.

In an embodiment, the water-soluble polymer is one or more selected from polyvinyl alcohol, polyethylene glycol, chitosan, hydroxymethyl cellulose, sodium carboxymethyl cellulose, and starch.

In an embodiment, the grafting reagent is one or more selected from N-(2,2-dimethoxymethyl)-2-acrylamide, N-(2,2-dimethoxyethyl)-2-acrylamide, and 4-acrylamidobutyraldehyde dimethyl acetal.

In an embodiment, a mass ratio of the water-soluble polymer to the modified polymer-containing solution is substantially (6.3 to 20.7):100.

In an embodiment, the water-soluble monomer further contains the ionic group, and the ionic group is one or two selected from a sulfonate group and a phosphate group.

In an embodiment, the unsaturated bonds each includes a carbon-carbon double bond or a carbon-carbon triple bond.

In an embodiment, the water-soluble initiator is one or more selected from ammonium persulfate, sodium persulfate, and potassium persulfate.

In an embodiment, the modified polymer is mixed in form of an aqueous solution, and a mass ratio of the modified polymer-containing solution, water, the water-soluble monomer, and the water-soluble initiator is substantially 150:(1 to 15):(3 to 50):(1 to 6), wherein the modified polymer-containing solution has a viscosity in a range from about 58 cps to about 1228 cps, and has an electrical conductivity in a range from about 7.6 µs/cm to about 48.9 µs/cm.

In an embodiment, the water-soluble monomer is one or more selected from sodium 2-acrylamido-2-methylpropanesulfonate, acrylamide, sodium acrylate, sodium methacrylate, and sodium allylsulfonate.

In an embodiment, a mass percentage concentration of the oil-soluble stabilizer in the oil phase solution is in a range from about 0.3% to about 1.7%.

In an embodiment, the oily solvent is one or more selected from n-butyl acetate, ethyl acetate, methyl acetate, and propyl acetate.

In an embodiment, the oil-soluble stabilizer is one or more selected from cellulose acetate butyrate, cellulose acetate, Span, and Tween.

In an embodiment, a volume ratio of the aqueous phase solution to the oil phase solution is substantially 1:(6 to 12).

In an embodiment, the method further includes: mixing the embolic microspheres with a contrast enhancing monomer, and performing a heating reaction under an action of an acid catalysis, wherein the contrast enhancing monomer is a mono-iodinated or poly-iodinated compound, and the contrast enhancing monomer further contains one or more groups selected from an aldehyde group, a carboxyl group, and a hydroxyl group.

In an embodiment, a mass ratio of the contrast enhancing monomer to the embolic microspheres is substantially (0.375 to 1.5):1.

In an embodiment, the contrast enhancing monomer is one or more selected from 3-acrylamido-2,4,6-triiodobenzoic acid, 3-acrylate-2,4,6-triiodobenzoic acid, 2,4,6-triiodobenzoic acid, 2,3,5-triiodobenzaldehyde, 2-iodobenzaldehyde, and 4-iodobenzaldehyde.

In an embodiment, the heating reaction is performed at a temperature in a range from about 40 °C to about 65 °C for a time period in a range from about 12 hours to about 24 hours.

In an embodiment, the method further includes filtering, washing, and sieving the reaction system after contrast enhancement to obtain contrast enhanced embolic microspheres with a particle size in a range from about 40 µm to about 90 µm.

Details of one or more embodiments of the present application are set forth in the accompanying drawings and description below. Other features, objectives, and advantages of the present application will become apparent from the description, drawings, and claims.

### BRIEF DESCRIPTION OF THE DRAWINGS

To illustrate the technical solutions in the embodiments of the present application or in related art more clearly, the drawings used in the embodiments or in the related art will be described briefly. Apparently, the following described drawings are merely for the embodiments of the present application, and other drawings can be derived according to the disclosed drawings by those of ordinary skill in the art without any creative effort.
FIG. 1 shows a flow chart of a method for preparing embolic microspheres in some embodiments of the present application.
FIG. 2 shows an optical microscopic image of embolic microspheres prepared under the conditions of Example 1.
FIG. 3 shows an optical microscopic image of embolic microspheres prepared under the conditions of Example 2.
FIG. 4 shows an optical microscopic image of embolic microspheres prepared under the conditions of Example 5.
FIG. 5 shows an optical microscopic image of embolic microspheres prepared under the conditions of Comparative Example 1.
FIG. 6 shows a particle size distribution diagram of the embolic microspheres prepared under the conditions of Example 1.
FIG. 7 shows a particle size distribution diagram of the embolic microspheres prepared under the conditions of Example 2.
FIG. 8 shows a particle size distribution diagram of the embolic microspheres prepared under the conditions of Comparative Example 1.
FIG. 9 shows a diagram comparing the yields of the embolic microspheres with a particle size in a range from 40 µm to 90 µm prepared in Example 1, Example 2, and Comparative Example 1.
FIG. 10 shows a postcontrast image of the embolic microspheres prepared under the conditions of Example 1 in the hepatic artery.
FIG. 11 shows a postcontrast image of the embolic microspheres prepared under the conditions of Example 2 in the hepatic artery.
FIG. 12 shows a postcontrast image of the embolic microspheres prepared under the conditions of Comparative Example 1 in the hepatic artery.
FIG. 13 shows an optical microscopic image of the embolic microspheres prepared under the conditions of Comparative Example 2.
FIG. 14 shows an optical microscopic image of the embolic microspheres prepared under the conditions of Comparative Example 3.
FIG. 15 shows an optical microscopic image of the embolic microspheres prepared under the conditions of Comparative Example 5.
FIG. 16 shows an optical microscopic image of the embolic microspheres prepared under the conditions of Comparative Example 6.

### DETAILED DESCRIPTION

The technical solutions of the embodiments of the present application will be described more clearly and comprehensively below in conjunction with the accompanying drawings for the embodiments of the present application. Apparently, the embodiments described herein are only part of, not all of the embodiments of the present application. Base on the embodiments of the present application, other embodiments obtained by those of ordinary skill in the art without creative efforts shall fall within the protection scope of the present application.

As described in the background, compared with larger sized embolic microspheres, the small-sized embolic microspheres can induce more extensive and significant tumor necrosis, embolize more distal tumor tissues, and thereby reduce damage of the microspheres to normal tissues. However, the conventional preparation method for the small-sized embolic microspheres suffers from issues such as low yield and poor appearance. In view of this, the present application provides a method that can improve the yield while maintaining good appearance of the small-sized embolic microspheres without compromising the microspheres' performance. This method offers more product options for clinical procedures, achieves better embolization effects, and further enhances the therapeutic efficacy of interventional embolization.

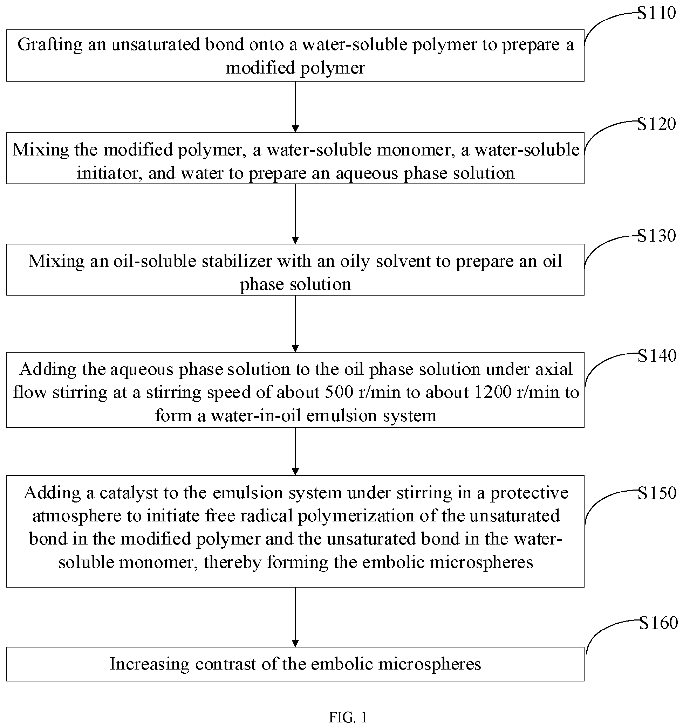

Referring to FIG. 1, in some embodiments of the present application, a method for preparing an embolic microsphere includes the following steps:
Step S110: grafting an unsaturated bond onto a water-soluble polymer to prepare a modified polymer.

In some embodiments, the water-soluble polymer contains a hydroxyl group. In an embodiment, the water-soluble polymer is at least one selected from polyvinyl alcohol, polyethylene glycol, chitosan, carboxymethyl cellulose, sodium carboxymethyl cellulose, and starch. Further, the water-soluble polymer is polyvinyl alcohol.

In some embodiments, a grafting reagent contains an acetal group and the unsaturated bond. In some embodiment, step S110 includes reacting the water-soluble polymer with the grafting reagent containing the acetal group and the unsaturated bond under an acidic condition to prepare the modified polymer.

In an embodiment, the grafting reagent is one or more selected from N-(2,2-dimethoxymethyl)-2-acrylamide, N-(2,2-dimethoxyethyl)-2-acrylamide, and 4-acrylamidobutyraldehyde dimethyl acetal. Optionally, the grafting reagent is N-(2,2-dimethoxyethyl)-2-acrylamide.

In some embodiments, a mass ratio of the water-soluble polymer to the grafting reagent is substantially 1:(0.016 to 0.07). For example, the mass ratio of the water-soluble polymer to the grafting reagent can be, but is not limited to, 1:0.016, 1:0.02, 1:0.025, 1:0.03, 1:0.035, 1:0.04, 1:0.045, 1:0.05, 1:0.055, 1:0.06, 1:0.065, or 1:0.07, or can be in a range between any two of these values. An excessively small mass ratio of the grafting reagent to the water-soluble polymer may result in an excessively low crosslinking density of the embolic microspheres, thereby adversely affecting the appearance and mechanical performance of the microspheres. Conversely, an excessively great mass ratio of the grafting reagent to the water-soluble polymer may result in an excessively high crosslinking density of the embolic microspheres, thereby compromising the suspension and delivery performance of the microspheres. By controlling the mass ratio of the water-soluble polymer to the grafting reagent, the prepared small-sized embolic microspheres can achieve good appearance while maintaining favorable suspension and delivery performance.

In an embodiment, the temperature of the reaction is in a range from about 40 °C to about 50 °C, and the time period of the reaction is in a range from about 3 hours to about 24 hours.

In an embodiment, the acidic pH in step S110 is in a range from 1 to 2.

In an embodiment, an acidic reagent that is used is at least one selected from hydrochloric acid, sulfuric acid, nitric acid, and p-toluenesulfonic acid. In an embodiment, the acidic reagent is hydrochloric acid.

In some embodiments, a mass ratio of the water-soluble polymer to the modified polymer-containing solution is substantially (6.3 to 20.7):100. In an embodiment, the mass ratio of the water-soluble polymer to the modified polymer-containing solution can be, but is not limited to, 6.3:100, 7:100, 8:100, 9:100 ,10:100, 11:100, 12:100, 13:100, 14:100, 15:100, 16:100, 17:100, 18:100, 19:100, 20:100, or 20.7:100, or can be in a range between any two of these values.

In some embodiments, the modified polymer-containing solution has a viscosity in a range from about 58 cps to about 1228 cps, and has an electrical conductivity in a range from about 7.6 µs/cm to about 48.9 µs/cm.

Controlling the mass ratio of the modified polymer-containing solution to the water-soluble polymer can further improve the yield of the small-sized embolic microspheres. In practice, the mass ratio of the modified polymer-containing solution to the water-soluble polymer can be controlled by adjusting the dialysis process of the modified polymer-containing solution. While ensuring complete dialysis of small-molecule impurities, different mass ratios are achieved by extending or shortening the dialysis time.

In some embodiments, step S110 includes: dissolving the water-soluble polymer in water, and then adjusting pH to acidic, reacting the water-soluble polymer with the grafting reagent at a temperature in a range from about 40 °C to about 50 °C for a time period in a range from about 3 hours to about 24 hours, and then adjusting the pH to neutral or alkaline after the reaction, and performing dialysis purification to prepare the modified polymer-containing solution. The water-soluble polymer contains a hydroxyl group, and the grafting reagent contains an acetal group and the unsaturated bond. The mass ratio of the water-soluble polymer to the grafting reagent is substantially 1:(0.016 to 0.07). The mass ratio of the water-soluble polymer to the modified polymer-containing solution is substantially (6.3 to 20.7):100.

It should be understood that the step of dissolving the water-soluble polymer in water can be carried out using a common method in the art. For example, heating may be applied to accelerate dissolution. In an embodiment, when the water-soluble polymer is polyvinyl alcohol, the water-soluble polymer is first mixed with water and stirred, then heated to about 60 °C for swelling for about 45 minutes, followed by further heating to about 95 °C for dissolution for about 2 hours.

Step S120: mixing the modified polymer, a water-soluble monomer, a water-soluble initiator, and water to prepare an aqueous phase solution.

The water-soluble monomer contains an unsaturated bond. In some embodiments, the unsaturated bond is a carbon-carbon double bond or a carbon-carbon triple bond.

In some embodiments, the water-soluble monomer further contains an ionic group. The ionic group is one or more selected from a sulfonate group and a phosphate group. The water-soluble monomer containing the ionic group facilitates drug loading.

In some embodiments, the water-soluble monomer is one or more selected from sodium 2-acrylamido-2-methylpropane sulfonate, acrylamide, sodium acrylate, sodium methacrylate, and sodium allylsulfonate. Optionally, the water-soluble monomer is sodium 2-acrylamido-2-methylpropane sulfonate.

In some embodiments, the water-soluble initiator is a redox initiator. In an embodiment, the water-soluble initiator can be ammonium persulfate, sodium persulfate, potassium persulfate, or the like.

In some embodiments, the mass ratio of the modified polymer-containing solution, water, the water-soluble monomer, and the water-soluble initiator is substantially 150:(1 to 15):(3 to 50):(1 to 6).

In some embodiments, the mass ratio of the water-soluble polymer to the water-soluble monomer is substantially 1: (0.24 to 1.16). For example, the mass ratio of the water-soluble polymer to the water-soluble monomer can be, but is not limited to, 1:0.24, 1:0.3, 1:0.4, 1:0.5, 1:0.6, 1:0.7, 1:0.75, 1:0.8, 1:0.85, 1:0.9, 1:0.95, 1:1, 1:1.05, 1:1.1, or 1:1.16, or can be in a range between any two of these values. Controlling the mass ratio of the water-soluble monomer to the water-soluble polymer is beneficial to improving the appearance of the small-sized embolic microspheres.

Step S130: mixing an oil-soluble stabilizer with an oily solvent to prepare an oil phase solution.

In some embodiments, the oil-soluble stabilizer is one or more selected from a Span compound, a Tween compound, and a modified cellulose compound. For example, the Span compound can be Span 20, Span 80, etc. The Tween compound can be Tween 20, Tween 80, etc. The modified cellulose compound can be cellulose acetate, cellulose acetate butyrate. In an embodiment, the oil-soluble stabilizer is one or more selected from cellulose acetate butyrate, cellulose acetate, Span, and Tween. Optionally, the oil-soluble stabilizer is cellulose acetate butyrate.

In some embodiments, the oily solvent is one or more selected from n-butyl acetate, ethyl acetate, methyl acetate, and propyl acetate. In an embodiment, the oily solvent is n-butyl acetate.

In some embodiments, a mass percentage concentration of the oil-soluble stabilizer in the oil phase solution is in a range from about 0.3% to about 1.7%. For example, the mass percentage concentration of the oil-soluble stabilizer in the oil phase solution can be, but is not limited to, 0.3%, 0.4%%, 0.5%, 0.6%, 0.7%, 0.8%, 0.9%, 1%, 1.1%, 1.2%, 1.3%, 1.4%, 1.5%, 1.6%, or 1.7%, or can be in a range between any two of these values. The mass percentage concentration of the oil-soluble stabilizer within the above range is beneficial to improving the dispersion performance of the microspheres, preventing the microspheres from aggregation, while facilitating the subsequent treatment process.

Step S140: adding the aqueous phase solution to the oil phase solution under axial flow stirring at a stirring rotation speed in a range from about 500 r/min to about 1200 r/min to form a water-in-oil emulsion system.

In some embodiments, the stirring rotation speed is in a range from about 500 r/min to about 1200 r/min. For example, the stirring rotation speed can be, but is not limited to, 500 r/min, 600 r/min, 700 r/min, 800 r/min, 850 r/min, 900 r/min, 950 r/min, 1000 r/min, 1100 r/min, or 1200 r/min, or can be in a range between any two of these values. Optionally, the stirring rotation speed is in a range from 800 r/min to 1000 r/min. Optimizing the stirring rotation speed can improve the yield and appearance of the prepared embolic microspheres.

In some embodiments, the number of blades of a stirring paddle used in the axial flow stirring is greater than 2. Further, the stirring paddle includes a four-blade metal stirring paddle. By controlling the shape of the stirring paddle and the stirring rotation speed during the emulsification process, the yield of the prepared small-sized embolic microspheres can be increased.

In some embodiments, a volume ratio of the aqueous phase solution to the oil phase solution is substantially 1:(6 to 12). For example, the volume ratio of the aqueous phase solution to the oil phase solution can be, but is not limited to, 1:6, 1:7, 1:8, 1:9, 1:10, 1:11, or 1:12, or can be in a range between any two of these values. Optionally, the volume ratio of the aqueous phase solution to the oil phase solution is substantially 1:(8 to 12). Further, the volume ratio of the aqueous phase solution to the oil phase solution is substantially 1:(8 to 10). By controlling the volume ratio of the aqueous phase solution to the oil phase solution, the yield of small-sized embolic microspheres can be further increased.

In some embodiments, in step S140, the aqueous phase solution can be added dropwise to the oil phase solution at a dropping rate commonly used in the art.

Step S150: adding a catalyst to the emulsion system under stirring in a protective atmosphere to initiate free radical polymerization of the unsaturated bond of the modified polymer and the unsaturated bond of the water-soluble monomer, thereby forming the embolic microsphere.

In some embodiments, the catalyst is tetramethylethylenediamine, a persulfate, or a combination thereof. Optionally, the catalyst is tetramethylethylenediamine. Tetramethylethylenediamine is used to catalyze ammonium persulfate to generate free radicals, which initiate the free radical polymerization. In another embodiment, the catalyst includes a primary catalyst and a co-catalyst, where the primary catalyst is tetramethylethylenediamine, and the co-catalyst is one or two persulfates.

In some embodiments, a mass ratio of the catalyst to the modified polymer is substantially (0.533 to 4.8):100. For example, a mass ratio of the catalyst to the modified polymer can be, but is not limited to, 0.533:100, 0.8:100, 1:100, 1.5:100, 2:100, 2.5:100, 3:100, 3.5:100, 4:100, 4.5:100, or 4.8:100, or can be in a range between any two of these values.

In some embodiments, the stirring rotation speed in step S140 is in a range from about 150 r/min to about 400 r/min. Further, the stirring paddle used in step S140 is a two-blade stirring paddle.

In some embodiments, the temperature for the free radical polymerization is in a range from about 45 °C to about 65 °C. For example, the temperature for the free radical polymerization can be, but is not limited to, 45 °C, 48 °C, 50 °C, 52 °C, 55 °C, 56 °C, 58 °C, 60 °C, 62 °C, or 65 °C, or in a range between any two of these values.

In some embodiments, the duration of the free radical polymerization is in a range from about 2 hours to about 8 hours. For example, the duration of the free radical polymerization can be, but is not limited to, 2 hours, 3 hours, 4 hours, 5 hours, 6 hours, 7 hours, or 8 hours, or can be in a range between any two of these values.

In some embodiments, after the free radical polymerization, the method further includes filtering, washing, and drying the reaction product. In an embodiment, the reaction product is settled, the supernatant is decanted, and the reaction product is then filtered, washed with n-butyl acetate and acetone, and dried.

In some embodiments, step S140 and step S150 are performed in two separate devices. For example, step S140 is carried out in a beaker, while step S150 is carried out in a reactor. A polymerization reaction is typically carried out in a reactor. However, due to the limitations of current technology, the maximum stirring rotation speed of the polymerization reactor is 550 r/min, and a two-blade stirring paddle is commonly used, which cannot adequately satisfy the requirements of the emulsification process. By conducting the polymerization and the emulsification in two separate devices, the stirring rotation speed and paddle shape for the emulsification process can be independently adjusted, not affected by the speed range of the reactor, thereby improving the yield of the small-sized embolic microspheres. Moreover, conducting the emulsification and the polymerization in separate devices enables an increased batch size, thus enhancing the production efficiency of the prepared embolic microspheres.

Step S160: enhancing a contrast of the embolic microspheres.

In some embodiments, step S160 includes: mixing the embolic microsphere with a contrast enhancing monomer, and performing a heating reaction under an action of an acid catalysis, wherein the contrast enhancing monomer is a mono-iodinated or poly-iodinated compound, and the contrast enhancing monomer further contains a functional group capable of reacting with the embolic microsphere.

In an embodiment, the contrast enhancing monomer is the mono-iodinated or poly-iodinated compound containing one or more groups selected from an aldehyde group, a carboxyl group, and a hydroxyl group. In an embodiment, the contrast enhancing monomer is one or more selected from 3-acrylamido-2,4,6-triiodobenzoic acid, 3-acrylate-2,4,6-triiodobenzoic acid, 2,4,6-triiodobenzoic acid, 2,3,5-triiodobenzaldehyde, 2-iodobenzaldehyde, and 4-iodobenzaldehyde. Optionally, the contrast enhancing monomer is 2,3,5-triiodobenzaldehyde.

In some embodiments, a mass ratio of the contrast enhancing monomer to the embolic microspheres is substantially (0.375 to 1.5):1. For example, the mass ratio of the contrast enhancing monomer to the embolic microspheres can be 0.375:1, 0.4:1, 0.5:1, 0.6:1, 0.7:1, 0.8:1, 0.9:1, 1:1, 1.2:1, 1.4:1, or 1.5:1, or can be in a range between any two of these values.

In some embodiments, the temperature for the heating reaction is in a range from about 40 °C to about 65 °C. For example, the temperature for the heating reaction can be, but is not limited to, 40 °C, 42 °C, 45 °C, 48 °C, 50 °C, 52 °C, 55 °C, 56 °C, 58 °C, 60 °C, 62 °C, or 65 °C, or in a range between any two of these values.

In some embodiments, the duration of the heating reaction is in a range from about 12 hours to about 24 hours. For example, the duration of the heating reaction can be, but is not limited to, 12 hours, 14 hours, 16 hours, 18 hours, 20 hours, 22 hours, or 24 hours, or in a range between any two of these values.

In some embodiments, step S160 includes: introducing a solution containing the contrast enhancing monomer into a solution containing the embolic microspheres, adding an acid catalyst, and performing the heating reaction at about 40 °C to about 65 °C for about 12 hours to about 24 hours.

In an embodiment, in the solution containing the contrast enhancing monomer, the solvent is dimethyl sulfoxide. In the solution containing the embolic microspheres, the solvent is dimethyl sulfoxide.

In an embodiment, the acid catalyst is methanesulfonic acid. It should be understood that, the acid catalyst is not limited to this and can be other acidic reagents.

In some embodiments, after the heating reaction, the method further includes filtering, washing, and sieving the reaction system.

It should be understood that, although the steps in the flow chart shown in FIG. 1 are displayed sequentially according to the direction of the arrows, these steps are not necessarily performed in the order indicated by the arrows. Unless otherwise specified herein, the sequence of these steps is not strictly limited, and these steps can also be performed in other orders. For example, step S130 can be performed before step S110 or step S120, or can be performed simultaneously with step S110 or step S120. Furthermore, at least some of the steps in FIG. 1 can include multiple sub-steps or multiple stages. These sub-steps or stages are not necessarily performed at the same time, but can be performed at different times. These sub-steps or stages are not necessarily to be sequentially performed, but can be performed alternately or in turn with at least some of the other steps, sub-steps, or stages.

In the clinical procedure of interventional embolization, a physician first needs to mix the embolic agent with a contrast agent, and then deliver the embolic material to the lesion site via a microcatheter. The most challenging aspect throughout the entire process lies in determining the embolization endpoint. Since the precise location of the embolic agent cannot be confirmed, the physician can only indirectly assess the embolization endpoint by observing the cessation of blood flow in the target vessel injected with the contrast agent, which may lead to erroneous embolization or incomplete embolization, posing a significant risk and adversely affecting the treatment effect. Therefore, in the present embodiment, the embolic microspheres can be subjected to contrast enhancement, increasing the contrast of the embolic microspheres, which meets higher clinical requirements for embolic microspheres and is of great significance for promoting the clinical application of embolic microspheres.

Imparting enhanced contrast to the embolic microspheres may increase their density and decrease their water content, which can deteriorate their suspension performance, thereby affecting their ability to smoothly pass through the microcatheter. To compensate for the loss of water content and density of the microspheres, the amount of water-soluble monomer has to be increased to enhance the hydrophilicity of the microspheres. However, an excessive amount of water-soluble monomer may degrade the appearance of the microspheres. Consequently, to address the appearance degradation issue of the microspheres, the amount of the grafting reagent has to be increased to increase the crosslinking density of the microspheres. Therefore, to maintain the performance of the embolic microspheres while improving the contrast, the amounts of both the grafting reagent and the water-soluble monomer are to be increased upon the original embolic microsphere formulation.

There are several conventional methods for preparing small-sized embolic microspheres: One is the microfluidic method, which suffers from low preparation efficiency and is difficult to industrialize. The second involves simply increasing the stirring speed on the existing embolic microsphere preparation methods. However, this method tends to generate bubbles during the sphere-forming process after emulsification, yielding microspheres with poor appearance, thereby adversely affecting the deliverability performance and embolization performance of the microspheres. The third is electrostatic spraying, which is limited by solution concentration.

The method for preparing embolic microspheres according to the present embodiments offers at least the following advantages:
(1) In the method for preparing embolic microspheres, the modified polymer grafted with the unsaturated bond, the water-soluble monomer, and the water-soluble initiator are mixed to prepare the aqueous phase solution first. Then, under the control of stirring speed and paddle shape, the aqueous phase solution is added to the oil phase solution for emulsification. Finally, the catalyst is added to the emulsion system to initiate the free radical polymerization between the unsaturated bond of the modified polymer and the unsaturated bond of the water-soluble monomer, thereby forming the embolic microspheres. Based on the conventional embolic microsphere preparation process, the stirring rotation speed and paddle shape used in emulsification are adjusted to increase the yield of small-sized embolic microspheres. Moreover, by controlling the mass ratios of the water-soluble polymer to the grafting reagent and to the water-soluble monomer, the resulting small-sized embolic microspheres exhibit good appearance, regular shape, uniform size, and narrow size distribution, demonstrating superior suspension and deliverability performances, thereby achieving enhanced embolization efficacy to meet higher and broader clinical requirements.
(2) In the method for preparing embolic microspheres, the emulsification and polymerization processes can be separated and conducted in independent devices. By using a simple, separate emulsification device, the emulsification process is not limited by the speed range of the reactor, which enables batch production and significantly improves the production efficiency of the small-sized embolic microspheres.
(3) The method for preparing embolic microspheres can further increase the contrast of the embolic microspheres, which meets higher clinical requirements for embolic microspheres and is of great significance for promoting the clinical application of embolic microspheres.
(4) The method for preparing embolic microspheres is adjusted and optimized on the basis of the conventional embolic microsphere preparation process, maintaining simplicity without increasing process complexity. Compared with the microfluidic method, the present method for preparing embolic microspheres is simpler, more efficient, and easier to industrialize. Compared with the electrostatic spraying, the present method is not limited by solution concentration.

In order to further clarify the objectives and advantages of the present application, the embolic microspheres of the present application and the effects thereof will be further described in detail in conjunction with the specific embodiments below. It should be understood that, the specific embodiments described herein are for the purpose of explaining the present application and are not intended to limit the present application. Unless otherwise specified, the following embodiments do not include other components except for unavoidable impurities. Unless otherwise stated, the drugs and instruments used in the embodiments are conventional choices in the art. The experimental methods for which specific conditions are not specified are carried out under conventional conditions in the embodiments, such as those described in the literature, books, or methods recommended by the manufacturers.

### Example 1

This example provides a method for preparing embolic microspheres, including the following steps:
(1) Preparation of modified polymer: 150 mL of purified water was added to a 500 mL three-neck flask. Then, 50 g of polyvinyl alcohol with a molecular weight of 22,000 to 26,000 was added under stirring at room temperature, heated to 60 °C for swelling for 45 minutes, and then heated to 95 °C for dissolution for 2 hours. After the polyvinyl alcohol was dissolved completely, the resulting system was cooled to room temperature, and 1.7 g of N-(2,2-dimethoxyethyl)-2-acrylamide was added and then stirred for 15 minutes. Next, concentrated hydrochloric acid as the catalyst was added dropwise to adjust the pH of the reaction system to 1 to 2. After that, the temperature was raised to 40 °C for reaction for another 3 hours. After the reaction was completed, the pH of the reaction system was adjusted to 7 to 8 using a 4 mol/L sodium hydroxide solution, and the reaction system was then stabilized for 15 minutes. The crude product was collected and subjected to dialysis purification overnight using a dialysis bag until the dialysate had an electrical conductivity of less than 50 µs/cm and had a viscosity of 117 cps, yielding 400 g of a solution containing modified polyvinyl alcohol. The mass ratio of polyvinyl alcohol to the modified polyvinyl alcohol solution was 12.5%.
(2) Preparation of aqueous phase solution: 0.8 g of ammonium persulfate, 5 g of sodium 2-acrylamido-2-methylpropanesulfonate, and 5 g of purified water were successively added to a 25 mL beaker, stirred at room temperature until the ammonium persulfate and the sodium 2-acrylamido-2-methylpropanesulfonate were dissolved completely. Then, under stirring, the resulting mixture was slowly added to a beaker containing 100 g of the modified polyvinyl alcohol solution prepared in step (1) and evenly mixed, obtaining the aqueous phase solution for later use.
(3) Preparation of oil phase solution: 800 mL of n-butyl acetate solvent and 11.5 g of cellulose acetate butyrate stabilizer were added to a 2000 mL beaker, and stirred using a four-blade metal stirring paddle. The rotation speed of the mechanical stirrer was adjusted to 800 r/min. The mixture was stirred until the cellulose acetate butyrate was completely dissolved, obtaining the oil phase solution for later use.
(4) Emulsification: A four-blade metal stirring paddle was used, and the rotation speed of the mechanical stirrer was adjusted to 800 r/min. Under stirring at room temperature, the aqueous phase solution prepared in step (2) was added dropwise to the beaker containing the oil phase solution prepared in step (3), forming a water-in-oil emulsion system with a volume ratio of oil to water of 8:1.
(5) Polymerization: Under nitrogen protection at room temperature, the emulsion system obtained in step (4) was added to a 2000 mL reactor equipped with a two-blade polytetrafluoroethylene stirring paddle. The rotation speed was adjusted to 200 r/min.1.6 mL of tetraethylethylenediamine was added, and the system was heated to a temperature of 55 °C for reaction for 3 hours. After the reaction, the reaction solution was settled completely, followed by removing the supernatant. The reaction product was filtered, washed with n-butyl acetate and acetone, and then dried, yielding 13.4 g of dry embolic microspheres.
(6) Contrast enhancement: Under nitrogen protection, 5 g of the dry embolic microspheres obtained in step (5) was introduced into a 500 mL four-neck flask containing 250 mL of dimethyl sulfoxide and swelled fully at 50 °C for 4 hours. 3.75 g of the contrast enhancing monomer 2,3,5-triiodobenzaldehyde was completely dissolved in 20 mL of dimethyl sulfoxide, then added dropwise into the swollen microsphere system and stirred for 15 minutes for uniform mixing. 20 mL of methanesulfonic acid was added dropwise to the reaction system and then reacted at 50 °C for 20 hours. After the reaction, the reaction solution was filtered, washed with dimethyl sulfoxide and water, and sieved to obtain contrast enhanced embolic microspheres with different particle sizes. The yield of microspheres with a particle size of 40 µm to 90 µm was 37%.

### Example 2

This example provides a method for preparing embolic microspheres, including the following steps:
(1) Preparation of modified polymer: 200 mL of purified water was added to a 500 mL three-neck flask. Then, 50 g of polyvinyl alcohol with a molecular weight of 22,000 to 26,000 was added under stirring at room temperature, heated to 60 °C for swelling for 45 minutes, and then heated to 95 °C for dissolution for 2 hours. After the polyvinyl alcohol was dissolved completely, the resulting system was cooled to room temperature, and 2 g of N-(2,2-dimethoxyethyl)-2-acrylamide was added and then stirred for 15 minutes. Next, concentrated hydrochloric acid was added dropwise to adjust the pH of the reaction system to 1 to 2. After that, the temperature was raised to 40 °C for reaction for another 6 hours. After the reaction was completed, the pH of the reaction system was adjusted to 7 to 8 using a 4 mol/L sodium hydroxide solution, and the reaction system was then stabilized for 15 minutes. The crude product was collected and subjected to dialysis purification overnight using a dialysis bag until the dialysate had an electrical conductivity of less than 50 µs/cm and had a viscosity of 74 cps, yielding 380 g of a solution containing modified polyvinyl alcohol. The mass ratio of polyvinyl alcohol to the modified polyvinyl alcohol solution was 13.2%.
(2) Preparation of aqueous phase solution: 1.2 g of ammonium persulfate, 7.5 g of sodium 2-acrylamido-2-methylpropanesulfonate, and 7.5 g of purified water were successively added to a 25 mL beaker, stirred at room temperature until the ammonium persulfate and the sodium 2-acrylamido-2-methylpropanesulfonate were dissolved completely. Then, under stirring, the resulting mixture was slowly added to a beaker containing 150 g of the modified polyvinyl alcohol solution prepared in step (1) and evenly mixed, obtaining the aqueous phase solution for later use.
(3) Preparation of oil phase solution: 1400 mL of n-butyl acetate solvent and 17.25 g of cellulose acetate butyrate stabilizer were added to a 2000 mL beaker, and stirred using a four-blade metal stirring paddle. The rotation speed of the mechanical stirrer was adjusted to 800 r/min. The mixture was stirred until the cellulose acetate butyrate was completely dissolved, obtaining the oil phase solution for later use.
(4) Emulsification: A four-blade metal stirring paddle was used, and the rotation speed of the mechanical stirrer was adjusted to 900 r/min. Under stirring at room temperature, the aqueous phase solution prepared in step (2) was added dropwise to the beaker containing the oil phase solution prepared in step (3), forming a water-in-oil emulsion system with a volume ratio of oil to water of 9.3:1.
(5) Polymerization: Under nitrogen protection at room temperature, the emulsion system obtained in step (4) was added to a 2000 mL reactor equipped with a two-blade polytetrafluoroethylene stirring paddle. The rotation speed was adjusted to 200 r/min.2.4 mL of tetraethylethylenediamine was added, and the system was heated to a temperature of 55 °C for reaction for 5 hours. After the reaction, the initial reaction solution was settled completely, followed by removing the supernatant. The reaction product was filtered, washed with n-butyl acetate and acetone, and then dried, yielding 19.8 g of dry embolic microspheres.
(6) Contrast enhancement: Under nitrogen protection, 10 g of the dry embolic microspheres obtained in step (5) was introduced into a 1000 mL four-neck flask containing 500 mL of dimethyl sulfoxide and swelled fully at 50 °C for 4 hours. 7.5 g of the contrast enhancing monomer 2,3,5-triiodobenzaldehyde was completely dissolved in 40 mL of dimethyl sulfoxide, then added dropwise into the swollen microsphere system and stirred for 15 minutes for uniform mixing. 40 mL of methanesulfonic acid was added dropwise to the reaction system and then reacted at 50 °C for 20 hours. After the reaction, the initial reaction solution was filtered, washed with dimethyl sulfoxide and water, and sieved to obtain contrast enhanced embolic microspheres with different particle sizes. The yield of embolic microspheres with a particle size of 40 µm to 90 µm was 41%.

### Example 3

This example provides a method for preparing embolic microspheres with steps similar to those in Example 2. The difference lies in that: in step (3) of this example, the volume of the n-butyl acetate solvent was 800 mL. Consequently, in step (4), the volume ratio of oil to water was 5.3:1. Finally, the yield of the prepared embolic microspheres with a particle size of 40 µm to 90 µm was 29%.

### Example 4

This example provides a method for preparing embolic microspheres with steps similar to those in Example 2. The difference lies in that: in step (1) of this example, the dialysis purification was performed until the dialysate had an electrical conductivity of less than 50 µs/cm and a viscosity of 59.7 cps, yielding 900 g of a solution containing modified polyvinyl alcohol. The mass ratio of polyvinyl alcohol to the solution containing modified polyvinyl alcohol was 5.56:100.

In this example, the yield of the prepared embolic microspheres with a particle size of 40 µm to 90 µm was 39%.

### Example 5

This example provides a method for preparing embolic microspheres with steps similar to those in Example 2 except that: the rotation speed in step (4) was 1200 r/min.

In this example, the yield of the prepared embolic microspheres with a particle size of 40 µm to 90 µm was 45%.

### Example 6

This example provides a method for preparing embolic microspheres with steps similar to those in Example 2, except that: the rotation speed in step (4) was 500 r/min.

In this example, the yield of the prepared embolic microspheres with a particle size of 40 µm to 90 µm was 10%.

### Example 7

This example provides a method for preparing embolic microspheres with steps similar to those in Example 2 except that: in step (1), the mass of N-(2,2-dimethoxyethyl)-2-acrylamide was 0.8 g.

In this example, the yield of the prepared embolic microspheres with a particle size of 40 µm to 90 µm was 17%.

### Example 8

This example provides a method for preparing embolic microspheres with steps similar to those in Example 2, except that: in step (1), the mass of N-(2,2-dimethoxyethyl)-2-acrylamide was 3.5 g.

In this example, the yield of the prepared embolic microspheres with a particle size of 40 µm to 90 µm was 19%.

### Example 9

This example provides a method for preparing embolic microspheres with steps similar to those in Example 2, except that: in step (6), the mass of the contrast enhancing monomer 2,3,5-triiodobenzaldehyde was 17 g.

In this example, the yield of the prepared embolic microspheres with a particle size of 40 µm to 90 µm was 32%.

### Example 10

This example provides a method for preparing embolic microspheres with steps similar to those in Example 2, except that: the polymerization in step (5) was different. In this example, step (5) was as follows:
While maintaining the rotation speed at 900 r/min, 1.6 mL of tetraethylethylenediamine was added to the emulsion system obtained in step (4) under nitrogen protection at room temperature. The system was heated to a temperature of 55 °C for reaction for 3 hours. After the reaction, the reaction solution was settled completely, followed by removing the supernatant. The reaction product was filtered, washed with n-butyl acetate and acetone, and then dried, yielding 13.4 g of dry embolic microspheres.

In this example, the yield of the prepared embolic microspheres with a particle size of 40 µm to 90 µm was 29%.

### Comparative Example 1

This comparative example provides a method for preparing embolic microspheres, including the following steps:
(1) Preparation of modified polymer: 200 mL of purified water was added to a 500 mL three-neck flask. Then, 50 g of polyvinyl alcohol with a molecular weight of 22,000 to 26,000 was added under stirring at room temperature, heated to 60 °C for swelling for 45 minutes, and then heated to 95 °C for dissolution for 2 hours. After the polyvinyl alcohol was dissolved completely, the resulting system was cooled to room temperature, and 1.7 g of N-(2,2-dimethoxyethyl)-2-acrylamide was added and then stirred for 15 minutes. Next, concentrated hydrochloric acid as the catalyst was added dropwise to adjust the pH of the reaction system to 1 to 2. After that, the temperature was raised to 40 °C for reaction for another 3 hours. After the reaction was completed, the pH of the reaction system was adjusted to 7 to 8 using a 4 mol/L sodium hydroxide solution, and the reaction system was then stabilized for 15 minutes. The crude product was collected and subjected to dialysis purification overnight using a dialysis bag until the dialysate had an electrical conductivity of less than 50 µs/cm and had a viscosity of 97 cps, yielding 390 g of a solution containing modified polyvinyl alcohol. The mass ratio of polyvinyl alcohol to the modified polyvinyl alcohol solution was 12.8%.
(2) Preparation of aqueous phase solution: 0.8 g of ammonium persulfate, 5 g of sodium 2-acrylamido-2-methylpropanesulfonate, and 5 g of purified water were successively added to a 25 mL beaker, stirred at room temperature until the ammonium persulfate and the sodium 2-acrylamido-2-methylpropanesulfonate were dissolved completely. Then, under stirring, the resulting mixture was slowly added to a beaker containing 100 g of the modified polyvinyl alcohol solution prepared in step (1) and evenly mixed, obtaining the aqueous phase solution for later use.
(3) Integrated operation of oil phase preparation, emulsification, and polymerization: 800 mL of n-butyl acetate solvent and 11.5 g of cellulose acetate butyrate stabilizer were added to a 2000 mL reactor, and stirred using a two-blade polytetrafluoroethylene stirring paddle. The rotation speed was adjusted to 550 r/min, which was the maximum speed of the reactor. The mixture was stirred until the cellulose acetate butyrate was completely dissolved, obtaining the oil phase solution. Under stirring at room temperature, the aqueous phase solution prepared in step (2) was added dropwise to the oil phase solution, forming a water-in-oil emulsion system. Under nitrogen protection at room temperature, 1.6 mL of tetraethylethylenediamine was added to the emulsion system, and the system was heated to a temperature of 55 °C for reaction for 3 hours. After the reaction, the reaction solution was settled completely, followed by removing the supernatant. The reaction product was filtered, washed with n-butyl acetate and acetone, and then dried, yielding 13.9 g of dry embolic microspheres.
(4) Contrast enhancement: Under nitrogen protection, 5 g of the dry embolic microspheres obtained in step (3) was introduced into a 500 mL four-neck flask containing 250 mL of dimethyl sulfoxide and swelled fully at 50 °C for 4 hours. 3.75 g of the contrast enhancing monomer 2,3,5-triiodobenzaldehyde was completely dissolved in 20 mL of dimethyl sulfoxide, then added dropwise into the swollen microsphere system and stirred for 15 minutes for uniform mixing. 20 mL of methanesulfonic acid was added dropwise to the reaction system and then reacted at 50 °C for 20 hours. After the reaction, the initial reaction solution was filtered, washed with dimethyl sulfoxide and water, and sieved to obtain contrast enhanced embolic microspheres with different particle sizes. The yield of embolic microspheres with a particle size of 40 µm to 90 µm was 7%.

By comparing Example 1, Example 2, and Example 3, it can be seen that the yield of embolic microspheres with the particle size of 40 µm to 90 µm prepared in Example 1 is 5 times higher than that in Comparative Example 1, while the yield of embolic microspheres with the particle size of 40 µm to 90 µm prepared in Example 2 is 6 times higher than that in Comparative Example 1.

### Comparative Example 2

This comparative example provides a method for preparing embolic microspheres, including the following steps:
(1) Preparation of modified polymer: 200 mL of purified water was added to a 500 mL three-neck flask. Then, 50 g of polyvinyl alcohol with a molecular weight of 22,000 to 26,000 was added under stirring at room temperature, heated to 60 °C for swelling for 45 minutes, and then heated to 95 °C for dissolution for 2 hours. After the polyvinyl alcohol was dissolved completely, the resulting system was cooled to room temperature, and 1.7 g of N-(2,2-dimethoxyethyl)-2-acrylamide was added and then stirred for 15 minutes. Next, concentrated hydrochloric acid as the catalyst was added dropwise to adjust the pH of the reaction system to 1 to 2. After that, the temperature was raised to 40 °C for reaction for another 3 hours. After the reaction was completed, the pH of the reaction system was adjusted to 7 to 8 using a 4 mol/L sodium hydroxide solution, and the reaction system was then stabilized for 15 minutes. The crude product was collected and subjected to dialysis purification overnight using a dialysis bag until the dialysate had an electrical conductivity of less than 50 µs/cm and had a viscosity of 78 cps, yielding 380 g of a solution containing modified polyvinyl alcohol. The mass ratio of polyvinyl alcohol to the modified polyvinyl alcohol solution was 13.2%.
(2) Preparation of aqueous phase solution: 0.4 g of ammonium persulfate, 5 g of sodium 2-acrylamido-2-methylpropanesulfonate, and 5 g of purified water were successively added to a 25 mL beaker, stirred at room temperature until the ammonium persulfate and the sodium 2-acrylamido-2-methylpropanesulfonate were dissolved completely. Then, under stirring, the resulting mixture was slowly added to a beaker containing 50 g of the modified polyvinyl alcohol solution prepared in step (1) and evenly mixed, obtaining the aqueous phase solution, for later use.
(3) Integrated operation of oil phase preparation, emulsification, and polymerization: 300 mL of n-butyl acetate solvent and 5.75 g of cellulose acetate butyrate stabilizer were added to a 1000 mL beaker, and stirred using a two-blade polytetrafluoroethylene stirring paddle. The rotation speed of the mechanical stirrer was adjusted to 800 r/min. The mixture was stirred until the cellulose acetate butyrate was completely dissolved, obtaining the oil phase solution for later use. Under stirring at room temperature, the aqueous phase solution prepared in step (2) was added dropwise to the oil phase solution, forming a water-in-oil emulsion system. Under nitrogen protection at room temperature, 1.6 mL of tetraethylethylenediamine was added to the emulsion system, and the system was heated to a temperature of 55 °C for reaction for 3 hours. After the reaction, the reaction solution was settled completely, followed by removing the supernatant. The reaction product was filtered, washed with n-butyl acetate and acetone, and then dried, yielding 6.3 g of dry embolic microspheres.
(4) Contrast enhancement: Under nitrogen protection, 5 g of the dry embolic microspheres obtained in step (3) was introduced into a 500 mL four-neck flask containing 250 mL of dimethyl sulfoxide and swelled fully at 50 °C for 4 hours. 3.75 g of the contrast enhancing monomer 2,3,5-triiodobenzaldehyde was completely dissolved in 20 mL of dimethyl sulfoxide, then added dropwise into the swollen microsphere system and stirred for 15 minutes for uniform mixing. 20 mL of methanesulfonic acid was added dropwise to the reaction system and then reacted at 50 °C for 20 hours. After the reaction, the reaction solution was filtered, washed with dimethyl sulfoxide and water, and sieved to obtain contrast enhanced embolic microspheres with different particle sizes. The yield of the microspheres with a particle size of 40 µm to 90 µm was 27%.

### Comparative Example 3

Comparative Example 3 provides a method for preparing embolic microspheres with steps similar to those in Example 2. The difference lies in that: in this comparative example, during the preparation of the aqueous phase solution in step (2), the mass of sodium 2-acrylamido-2-methylpropanesulfonate was 25 g. In this comparative example, the mass ratio of sodium 2-acrylamido-2-methylpropanesulfonate to polyvinyl alcohol was 1.26: 1.

In this comparative example, the appearance of the microspheres after polymerization in step (5) was poor, and therefore the subsequent contrast enhancement step was not performed.

### Comparative Example 4

Comparative Example 4 provides a method for preparing embolic microspheres with steps similar to those in Example 2. The difference lies in that: in this comparative example, during the preparation of the aqueous phase solution in step (2), the mass of sodium 2-acrylamido-2-methylpropanesulfonate was 4 g. In this comparative example, the mass ratio of sodium 2-acrylamido-2-methylpropanesulfonate to polyvinyl alcohol was 0.2:1.

In this comparative example, the yield of the prepared embolic microspheres with a particle size of 40 µm to 90 µm was 5%.

### Comparative Example 5

Comparative Example 5 provides a method for preparing embolic microspheres with steps similar to those in Example 2. The difference lies in that: in this comparative example, during the preparation of modified polymer in step (1), the mass of N-(2,2-dimethoxyethyl)-2-acrylamide was 0.5 g. In this comparative example, the mass ratio of N-(2,2-dimethoxyethyl)-2-acrylamide to polyvinyl alcohol was 0.01:1.

In this comparative example, the yield of the prepared embolic microspheres with a particle size of 40 µm to 90 µm was 11%.

### Comparative Example 6

Comparative Example 6 provides a method for preparing embolic microspheres with steps similar to those in Example 2. The difference lies in that: in this comparative example, during the preparation of modified polymer in step (1), the mass of N-(2,2-dimethoxyethyl)-2-acrylamide was 4.0 g. In this comparative example, the mass ratio of N-(2,2-dimethoxyethyl)-2-acrylamide to polyvinyl alcohol was 0.08:1.

In this comparative example, the yield of the prepared embolic microspheres with a particle size of 40 µm to 90 µm was 13%.

### The following is the testing section:

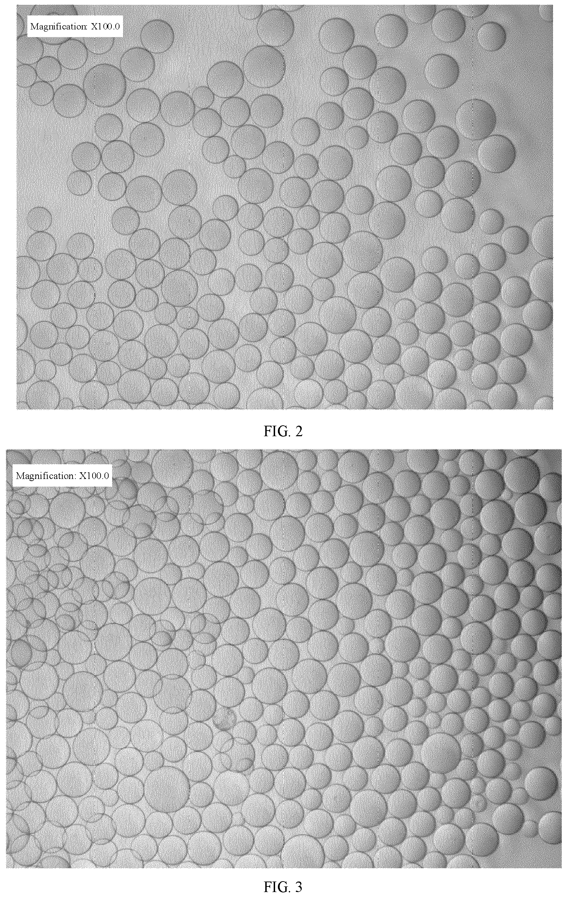

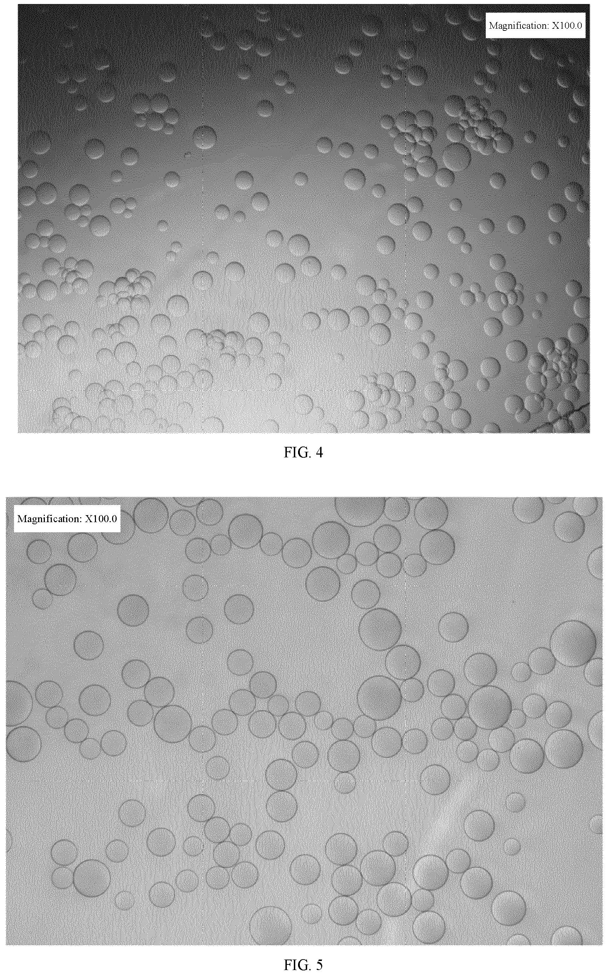

FIGs. 2, 3, and 5 show optical microscopic images of the contrast enhanced embolic microspheres prepared under the conditions of Example 1, Example 2, and Comparative Example 1, respectively. Compared with Comparative Example 1, the small-sized contrast enhanced embolic microspheres prepared in Examples 1 and 2 exhibit uniform particle size and regularly shaped spherical morphology. The embolic microspheres from Examples 3 to 4 and 6 to 10 also exhibit uniform particle size and regularly shaped spherical morphology, similar to those prepared in Examples 1 to 2. FIG. 4 shows an optical microscopic image of contrast enhanced embolic microspheres prepared under the conditions of Example 5, which also exhibit uniform particle size and regularly shaped spherical morphology. However, their appearance is slightly inferior to those of Examples 1 and 2 due to the increased rotation speed of 1200 r/min.

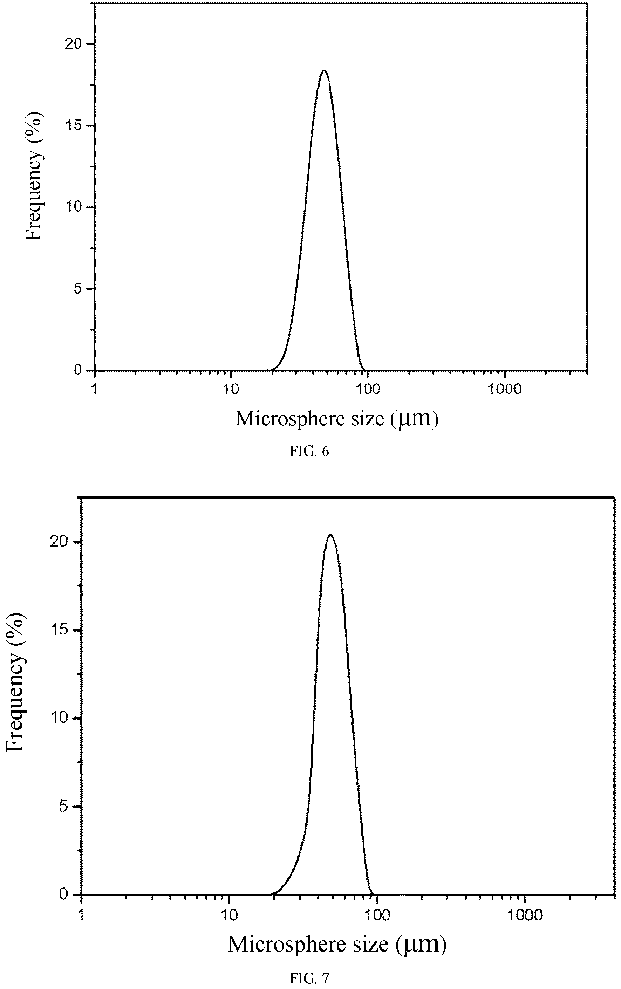

FIGs. 6, 7, and 8 show particle size distribution diagrams of the contrast enhanced embolic microspheres prepared under the conditions of Example 1, Example 2, and Comparative Example 1, respectively. It can be observed that the small-sized contrast enhanced embolic microspheres prepared in Examples 1 and 2, like those in Comparative Example 1, exhibit a narrow particle size distribution. However, the particle sizes of the embolic microspheres prepared in Examples 1 and 2 are significantly smaller than that of the embolic microspheres prepared in Comparative Example 1.

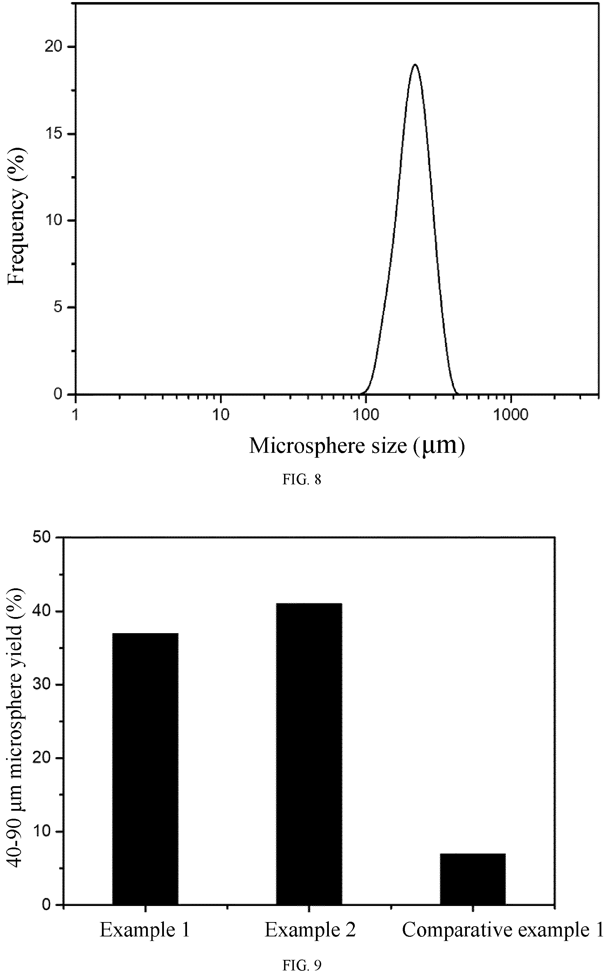

FIG. 9 shows a diagram comparing the yields of the embolic microspheres with a particle size in a range from 40 µm to 90 µm prepared in Example 1, Example 2, and Comparative Example 1. The result demonstrates that the optimized preparation process and formulation significantly improve the yield of the small-sized contrast enhanced embolic microspheres.

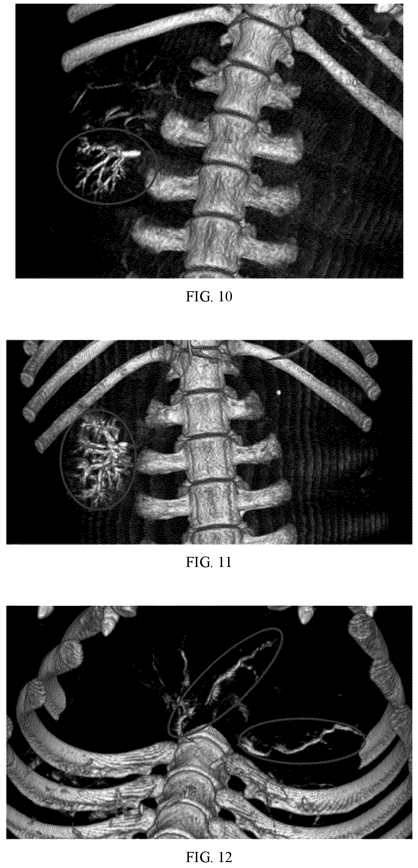

FIGs 10, 11, and 12 show postcontrast images of the embolic microspheres prepared under the conditions of Example 1, Example 2, and Comparative Example 1, in the hepatic artery, respectively. These images are CT scanning images taken 7 days after the embolic microspheres were injected into the hepatic artery of experimental pigs. All three images demonstrate clear contrast of the microspheres in the hepatic artery, confirming that the small-sized contrast enhanced microspheres prepared in Examples 1 and 2 exhibit *in vivo* contrast enhancement that is comparable or even superior to that of Comparative Example 1.

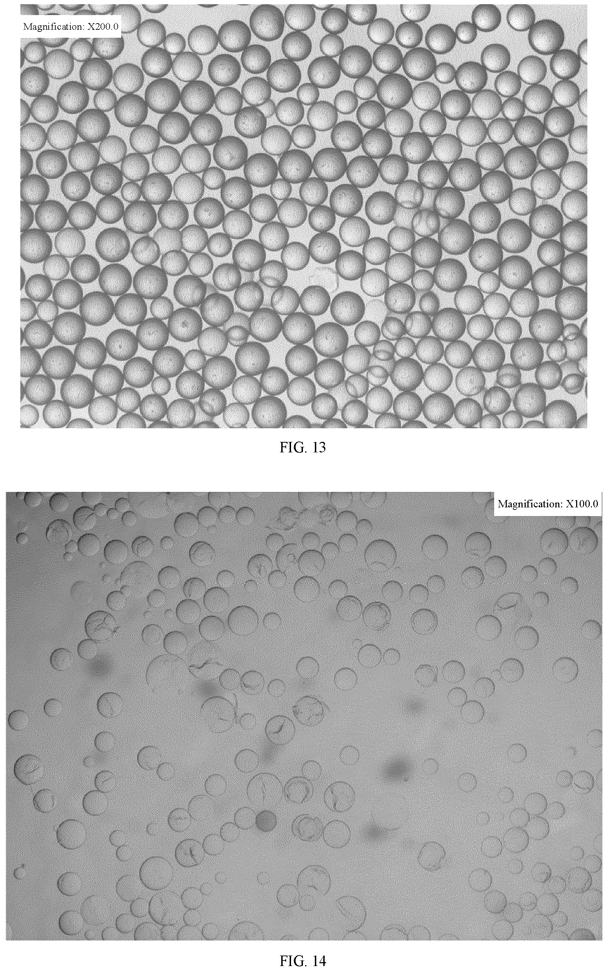

The appearance of the embolic microspheres prepared in Comparative Example 2 is shown in FIG. 13. It can be seen from the figure that the embolic microspheres prepared by this method exhibit an inferior appearance, with more bubbles and broken microspheres, compared to those in Examples 1 and 2.

The appearance of the embolic microspheres prepared in Comparative Example 3 is shown in FIG. 14. Due to the poor appearance of these embolic microspheres, no further contrast enhancement process was performed.

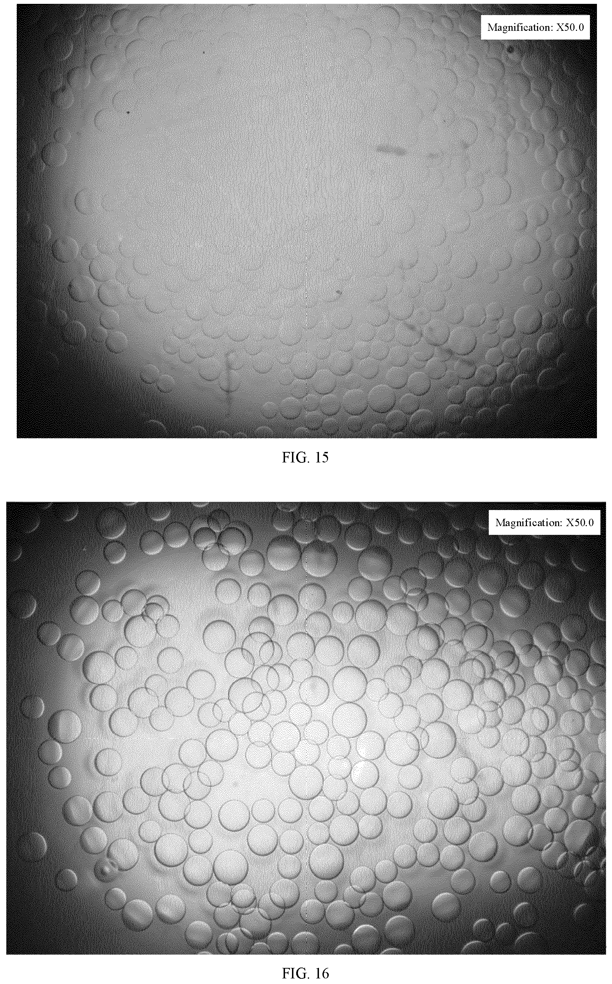

The appearance of the embolic microspheres prepared in Comparative Example 5 is shown in FIG. 15. Due to the high transparency of these embolic microspheres, which adversely affects their suspension and deliverability, no further contrast enhancement process was performed. Similarly, the embolic microspheres prepared in Comparative Example 4 also exhibit high transparency and have a similar appearance to those in Comparative Example 5, and thus their image was not redundantly provided.

The appearance of the embolic microspheres prepared in Comparative Example 6 is shown in FIG. 16. Due to the low transparency of these embolic microspheres, which adversely affects their suspension and deliverability, no further contrast enhancement process was performed.

Although the yields of the embolic microspheres prepared in Comparative Examples 5 and 6 are similar to those in Example 6, the poor transparency of the embolic microspheres prepared in Comparative Examples 5 and 6 adversely affects their suspension and deliverability. In contrast, the embolic microspheres prepared in Example 6 exhibit uniform particle size, regularly shaped spherical morphology, and excellent suspension and deliverability.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A method for preparing embolic microspheres, comprising following steps:
grafting an unsaturated bond onto a water-soluble polymer to prepare a modified polymer, wherein a mass ratio of the water-soluble polymer to a grafting reagent is substantially 1:(0.016 to 0.07), the water-soluble polymer comprises a hydroxyl group, and the grafting reagent comprises the unsaturated bond and an acetal group;
mixing the modified polymer, a water-soluble monomer, a water-soluble initiator, and water to prepare an aqueous phase solution, wherein the water-soluble monomer comprises an unsaturated bond and an ionic group, and a mass ratio of the water-soluble monomer to the water-soluble polymer is substantially (0.24 to 1.16):1;
mixing an oil-soluble stabilizer with an oily solvent to prepare an oil phase solution;
adding the aqueous phase solution to the oil phase solution under axial flow stirring at a stirring rotation speed in a range from about 500 r/min to about 1200 r/min to form a water-in-oil emulsion system; and
adding a catalyst to the emulsion system under stirring in a protective atmosphere to initiate free radical polymerization of the unsaturated bond of the modified polymer and the unsaturated bond of the water-soluble monomer, thereby forming the embolic microspheres.

2. The method according to claim 1, wherein in the step of adding the aqueous phase solution to the oil phase solution, the stirring rotation speed is in a range from about 800 r/min to about 1000 r/min.

3. The method according to claim 1 or 2, wherein in the step of adding the aqueous phase solution to the oil phase solution, a stirring paddle comprising a four-blade metal stirring paddle is adopted.

4. The method according to any one of claims 1 to 3, wherein a mass ratio of the catalyst to the modified polymer is substantially (0.533 to 4.8):100.

5. The method according to any one of claims 1 to 4, wherein the catalyst is tetramethylethylenediamine, a persulfate, or a combination thereof.

6. The method according to any one of claims 1 to 5, wherein the free radical polymerization is performed at a temperature in a range from about 45 °C to about 65 °C for a time period in a range from about 2 hours to about 8 hours.

7. The method according to any one of claims 1 to 6, wherein in the step of adding the catalyst to the emulsion system to initiate the free radical polymerization of the unsaturated bond of the modified polymer and the unsaturated bond of the water-soluble monomer, a stirring rotation speed is in a range from about 150 r/min to about 400 r/min.

8. The method according to any one of claims 1 to 7, wherein the step of grafting the unsaturated bond onto the water-soluble polymer comprises:
dissolving the water-soluble polymer in water and adjusting pH to acidic,
reacting the water-soluble polymer with the grafting reagent at a temperature in a range from about 40 °C to about 50 °C for a time period in a range from about 3 hours to about 24 hours and adjusting the pH to neutral or alkaline after the reaction, and
performing dialysis purification to prepare a modified polymer-containing solution.

9. The method according to claim 8, wherein a mass ratio of the water-soluble polymer to the modified polymer-containing solution is substantially (6.3 to 20.7):100.

10. The method according to any one of claims 1 to 9, wherein the water-soluble polymer is one or more selected from the group consisting of polyvinyl alcohol, polyethylene glycol, chitosan, hydroxymethyl cellulose, sodium carboxymethyl cellulose, and starch.

11. The method according to any one of claims 1 to 10, wherein the grafting reagent is one or more selected from the group consisting of N-(2,2-dimethoxymethyl)-2-acrylamide, N-(2,2-dimethoxyethyl)-2-acrylamide, and 4-acrylamidobutyraldehyde dimethyl acetal.

12. The method according to any one of claims 1 to 11, wherein the water-soluble monomer further contains the ionic group, and the ionic group is a sulfonate group, a phosphate group, or a combination thereof.

13. The method according to any one of claims 1 to 12, wherein the unsaturated bonds each comprises a carbon-carbon double bond or a carbon-carbon triple bond.

14. The method according to any one of claims 1 to 13, wherein the water-soluble initiator is one or more selected from the group consisting of ammonium persulfate, sodium persulfate, and potassium persulfate.

15. The method according to any one of claims 1 to 14, wherein the modified polymer is mixed in form of an aqueous solution, a mass ratio of the modified polymer-containing solution, water, the water-soluble monomer, the water-soluble initiator is substantially 150:(1 to 15):(3 to 50):(1 to 6), and the modified polymer-containing solution has a viscosity in a range from about 58 cps to about 1228 cps, and has an electrical conductivity in a range from about 7.6 µs/cm to about 48.9 µs/cm.

16. The method according to any one of claims 1 to 15, wherein the water-soluble monomer is one or more selected from the group consisting of sodium 2-acrylamido-2-methylpropanesulfonate, acrylamide, sodium acrylate, sodium methacrylate, and sodium allylsulfonate.

17. The method according to any one of claims 1 to 16, wherein a mass percentage concentration of the oil-soluble stabilizer in the oil phase solution is in a range from about 0.3% to about 1.7%.

18. The method according to any one of claims 1 to 17, wherein the oily solvent is one or more selected from n-butyl acetate, ethyl acetate, methyl acetate, and propyl acetate.

19. The method according to any one of claims 1 to 18, wherein the oil-soluble stabilizer is one or more selected from cellulose acetate butyrate, cellulose acetate, Span, and Tween.

20. The method according to any one of claims 1 to 19, wherein a volume ratio of the aqueous phase solution to the oil phase solution is substantially 1:(6 to 12).

21. The method according to any one of claims 1 to 20, further comprising:
mixing the embolic microspheres with a contrast enhancing monomer, and
performing a heating reaction under an action of an acid catalysis,
wherein the contrast enhancing monomer is a mono-iodinated compound or a poly-iodinated compound, and the contrast enhancing monomer further contains one or more groups selected from an aldehyde group, a carboxyl group, and a hydroxyl group.

22. The method according to claim 21, wherein a mass ratio of the contrast enhancing monomer to the embolic microspheres is substantially (0.375 to 1.5):1.

23. The method according to claim 21 or 22, wherein the contrast enhancing monomer is one or more selected from 3-acrylamido-2,4,6-triiodobenzoic acid, 3-acrylate-2,4,6-triiodobenzoic acid, 2,4,6-triiodobenzoic acid, 2,3,5-triiodobenzaldehyde, 2-iodobenzaldehyde, and 4-iodobenzaldehyde.

24. The method according to any one of claims 21 to 23, wherein the heating reaction is performed at a temperature in a range from about 40 °C to about 65 °C for a time period in a range from about 12 hours to about 24 hours.

25. The method according to any one of claims 21 to 24, further comprising: filtering, washing, and sieving the reaction system after contrast enhancement to obtain contrast enhanced embolic microspheres with a particle size in a range from about 40 µm to about 90 µm.
